# EUROPEAN PATENT SPECIFICATION

(11) **EP 0 610 446 B1**
(45) Date of publication and mention of the grant of the patent: **02.04.2003**
(21) Application number: 93908754.0
(22) Date of filing: 15.10.1992
(51) Int. Cl.: C07K 14/47, C07K 7/08, A61K 38/17, A61P 25/00, A61P 25/28

(54) **SYNTHETIC PEPTIDE SPECIFICITY OF ANTI-MYELIN BASIC PROTEIN FROM MULTIPLE SCLEROSIS CEREBROSPINAL FLUID**
SYNTHETISCHE PEPTIDE SPEZIFISCH GEGEN ANTI-BASISCHES-MYELIN-PROTEIN AUS CEREBROSPINALFLÜSSIGKEIT VON INDIVIDUEN MIT MULTIPLER SKLEROSE
SPECIFICITE DE PEPTIDES SYNTHETIQUES DE PROTEINES DE BASE ANTI-MYELINE PROVENANT DU LIQUIDE CEPHALO-RACHIDIEN D'UN INDIVIDU SOUFFRANT DE SCLEROSE MULTIPLE

(30) Priority: 22.10.1991 CA 2053799
(43) Date of publication of application: 17.08.1994
(73) Proprietor: The Governors of the University of Alberta, Edmonton, Alberta T6J 2J9 (CA)
(72) Inventor: WARREN, Kenneth G., Edmonton, Alberta T6H 3N7 (CA); CATZ, Ingrid, Edmonton, Alberta T6H 5N5 (CA)
(74) Representative: Stark, Amanda Jane
(86) International application number: CA9200448
(87) International publication number: WO93008212

(56) References cited:
- NATURE, Vol. 346, July 1990 Kohei Ota et al: "T-cell recognition of an immunodominant myelin basic protein epitope in multiple sclerosis ",
- Chemical Abstracts, volume 110, no. 1, 2 January 1989, (Columbus, Ohio, US), Dawkes, Adrian et al : "New monoclonal antibodies reactive with defined seqiential epitopes in human myelin basic protein ", see page 558, abstract 5882p, & J. Neuroimmunol. 1988, 19( 4), 305- 31
- Chemical Abstracts, volume 109, no. 19, 7 November 1988, (Columbus, Ohio, US), Katz, Jacqueline M. et al : "Antigenic and structural characterization of multiple subpopulations of H3N2 influenza virus form an individual ", see page 548, abstract 168486f, & Virology 1988, 165( 2), 446- 45
- Chemical Abstracts, volume 108, no. 19, 9 May 1988, (Columbus, Ohio, US), Higgins, Paul J. et al : "Suppression of experimental autoimmune encephalomyelitis by oral administration of myelin basic protein and its fragments ", see page 514, abstract 165933t, & J. Immunol. 1988, 140( 2), 440- 44
- Chemical Abstracts, volume 98, no. 13, 28 March 1983, (Columbus, Ohio, US), Gilliom, Richard D. et al : "Separation of myelin basic protein peptide 43-88 and its fragments by analytic and preparative high-performance liquid chromatography ", see page 304, abstract 103708q, & J. Chromatogr. 1983, 254(), 211- 21

## Description

This invention is concerned with the use of selected polypeptides in neutralizing antibodies to a human myelin protein. This invention also relates to a method of using these peptides for the treatment of Multiple Sclerosis.

### BACKGROUND AND PRIOR ART

Multiple sclerosis (MS) is a multifocal demyelinating disease of the human central nervous system (CNS) associated with inflammation. Increased intra-blood-brain barrier (intra-BBB) IgG synthesis is a hallmark of MS (Tourtelotte, W.W., J Neurol Sci 10: 279-304, 1970; Link, H. and Tibbling, G., Scand J Clin Lab Invest 37: 397-401, 1977; Tourtelotte, W.W. and Ma, B., Neurology 28: 76-83, 1978; Walsh, J.M. and Tourtelotte, W.W., In: Hallpike, J.F., Adams, C.W.M. and Tourtelotte, W.W., eds. Multiple sclerosis. Baltimore. Williams & Wilkins, 1982: 275-358; and Warren, K.G., and Catz, I. Ann Neurol 17: 475-480, 1985).

IgG synthesis within the BBB is generally elevated in clinically definite MS patients (Schumacher, G.A., Beebe, G., Kibler R.E., et al., Ann NY Acad Sci 15:266-272, 1965) with active or inactive disease. The specificity of the majority of the CNS IgG is unknown. While a small proportion has antiviral activity or reacts against brain antigens, nucleic acids, erythrocytes or smooth muscle antigens, the nonspecific portion may represent polyclonal activation of B-cells (Tourtelotte, W.W., and Ma, B., Neurology 28:76-83, 1978). During the last decade there has been considerable interest in the study of antibodies to specific myelin proteins.

Following the detection of circulating immune complexes containing myelin basic protein (MBP) as their antigenic component (Dasgupta, M.K., Catz, I, Warren, K.G. et al., Can J Neurol Sci 10:239-243, 1983), increased titers of antibodies to MBP (anti-MBP) were observed in the cerebrospinal fluid (CSF) of patients with active forms of MS (Warren, K.G. and Catz, I., Ann Neurol 209:20-25, 1986). Clinically, MS is characterized by phases of disease activity such as acute relapses or chronic progression, and by phases of clinical remission. Active MS is associated with increased levels of intrathecally produced anti-MBP (Warren, K.G. arid Catz, I., Ann Neurol 209:20-25, 1986; and Catz, I. and Warren, K.G., Can J Neurol Sci 13:21-24, 1986). These antibodies are found predominantly in free (F) form during acute relapses and predominantly in bound (B) form when the disease is insidiously progressive (Warren, K.G. and Catz, I., Ann Neurol 209:20-25, 1986). During acute relapses, CSF anti-MBP titers correlated with disease activity (Warren, K.G. and Catz, I., Ann Neurol 21:183-187, 1987). Anti-MBP levels were also found increased in patients with first attacks of optic neuritis and in most patients experiencing first attacks of MS (Warren, K.G., Catz, I., and Bauer, C., Ann Neurol 23:297-299, 1988; Warren, K.G. and Catz, I., J Neurol Sci 91:143-151, 1989).

Longitudinal kinetic studies of CSF anti-MBP levels in patients who enter the recovery phase subsequent to an acute relapse, demonstrated a gradual decline in F anti-MBP titers commensurate with a progressive rise in B fractions (Warren, K.G. and Catz, I., J Neurol Sci 91:143-151, 1989; Warren, K.G. and Catz, I., J Neurol Sci 88:185-194, 1988). In the remission phase, CSF anti-MBP may become undetectable suggesting an anti-MBP neutralization associated with inactive phases of MS (Warren, K.G. and Catz, I., J Neurol Sci 88:185-194, 1988). In contrast, chronic-progressive MS characterized by persistence of increased anti-MBP over long periods of time was associated with inhibition of anti-MBP neutralization (Warren, K.G. and Catz, I., J Neurol Sci 88:185-194, 1988). Recently a myelin basic protein antibody cascade, identified in the IgG fraction purified from CSF of MS patients, contained anti-MBP, antibodies which neutralize anti-MBP and antibodies which inhibit anti-MBP neutralization (Warren, K.G. and Catz, I., J Neurol Sci 96:19-27, 1990).

Ota et al in T-Cell Recognition of an Immunodominant Myelin Basic Protein Epitope in Multiple Sclerosis (Nature, Vol. 346, July 12, 1990) studies the T-cell specificity toward myelin basic protein, using T-cell lines established from multiple sclerosis patients.

PCT application published as WO-A-91/15225 recognizes the amino acid sequence from amino acid 82 to amino acid 104 as being an immunodominant domain of myelin basic protein, recognized by a majority of peripheral T-cells isolated from patients suffering from multiple sclerosis. This section of myelin basic protein is used to select potentially useful sections of T-cell chains and the nucleic acid sequences encoding those as potentially useful in therapies for multiple sclerosis.

Saikai, K. et al (PNAS, Vol 86 pg 9470-9474, 1989) describes peptides of myelin basic protein and the use of said peptide for the *in vivo* immunization of mice.

Groome, N. et al (Journal of Neuroimmunology, 19, 305-315, 1988) describes the production of various myelin basic protein peptides as well as their use *in vivo* for immunization of mice.

Price, J.O. et al (J. Of Immunology, Vol. 136, pg. 2426-2431, 1986) describes the presence of multiple epitopes of a decapeptide of myelin basic protein determined by monoclonal antibodies.

Vandenbark, A. A. et al (J. Of Immunology, Vol. 143, pg. 3512-3516, 1989) describes the determinant of myelin basic protein that induce encephalitogenic T-cells in Louis rats.

Previous studies have shown that anti-MBP is neutralized by MBP. However, previous attempts to treat MS by intramuscular or subcutaneous administration of heterologous MBP have not been successful (Campbel, B., Vogel, R.J., Fisher, E. and Lorenz, R., Arch Neurol 29:10-15, 1973; Gonsette, R.E., Delmotte, P. and Demonty, L., J Neurol 216:27-31, 1977; and Romine, J.S. and Salk, J., In: Hallpike, J.F., Adams, C.W.M. and Tourtelotte, W.W., eds. Multiple sclerosis. Baltimore. Williams & Wilkins, 1982:621-630). The problem with using native MBP is twofold. The protein is prepared from human brain samples and accordingly there is a potential danger that latent neuroviruses may be present in the sample. Secondly, although MBP is not normally an immunogen, it is possible that when administered to individuals with an altered immune system, MBP could act as an antigen and cause the production of antibodies against MBP.

Accordingly, an object of the present invention is to determine whether anti-MBP purified from CSF of MS patients with acute relapses could be neutralized by selected synthetic peptides of human MBP (h-MBP). For this purpose, synthetic peptides covering the entire length of h-MBP were used to determine the possible epitope range on h-MBP which neutralizes anti-MBP obtained from these patients. Therefore selected synthetic peptides, which demonstrate neutralization of anti-MBP, can be used to treat MS more effectively than the full length MBP. These synthetic peptides are non-naturally occurring and as such no potential threat of neuroviruses would exist. Additionally, due to their small size, these peptides could not act as an immunogen. Therefore, the use of selected synthetic peptides as a treatment for MS, would overcome the problems identified with using the native protein.

### SUMMARY OF INVENTION

The present invention provides for the use of a peptide having a length of from 8 amino acids to 25 amino acids, which peptide is substantially homologous in sequence to the amino acid sequence of a human myelin basic protein from amino acid 61 to amino acid 106 and is able to neutralize anti-myelin basic protein, in the manufacture of a medicament for the treatment of multiple sclerosis in humans, which medicament comprises the peptide as an active ingredient.

The present invention further provides a process for the manufacture of a medicament for the treatment of multiple sclerosis in a human, wherein said process comprises admixing a peptide having a length of from 8 amino acids to 25 amino acids, which peptide is substantially homologous in sequence to the amino acid sequence of a human myelin basic protein from amino acid 61 to amino acid 106 and is able to neutralize anti-myelin basic protein, with a suitable pharmaceutically acceptable carrier.

The present invention provides for the use of a human myelin protein peptide range having a length from 8 amino acids to 25 amino acids, which peptide is substantially homologous in sequence to the amino acid sequence of a human myelin basic protein from amino acid 61 to amino acid 106, in the manufacture of a medicament for the treatment of multiple sclerosis in humans, which medicament comprises the peptide as an active ingredient.

### BRIEF DESCRIPTION OF THE DRAWINGS

Fig. 1 shows the localization of eighteen synthetic peptides (small numbers) in relation to the intact human-MBP molecule. Peptides are represented by vertical bars placed next to their corresponding region on the MBP molecule. Large numbers represent amino acid residues on human MBP.

Fig. 2 demonstrates the neutralization of anti-MBP, purified and pooled from 10 different multiple sclerosis patients, by human MBP and 18 synthetic MBP-peptides.

Fig. 3 shows the neutralization of anti-MBP isolated from an individual multiple sclerosis patient by human MBP and peptides #12 (residues: 80-97), #15 (residues: 91-106) and #56 (residues: 75-95).

Fig. 4 depicts the entire amino acid sequence of human myelin basic protein, shown schematically in Fig. 1.

### DETAILED DESCRIPTION OF THE INVENTION

The present invention is directed to the use in the manufacture of a medicament of selected synthetic peptides, which are substantially homologous in sequence to a part of the amino acid sequence of the human myelin basic protein (h-MBP). By "substantially homologous" it is meant that some variation between the amino acid sequence of a human myelin protein, specifically h-MBP, and the synthetic peptides can exist provided that the synthetic peptides, with a variation in amino acid sequence, still function in their intended use, i.e. to neutralize antibodies to human myelin proteins or h-MBP (anti-MBP). Given the teachings of the present invention, it would be readily apparent to persons skilled in the art to determine, empirically, what variation can be made to the sequence of the selected peptides without affecting the function of the peptides.

The compounds used according to the present invention can be prepared according to conventional and well-known methods of synthesizing polypeptides. Also included within the scope of the term "synthetic peptide" are peptides produced from controlled hydrolysis of the naturally occurring myelin proteins to produce the selected synthetic peptides of the present invention. Also included within the scope of the term "synthetic peptide" are peptides produced by recombinant DNA technology. Knowing the sequence of the selected synthetic peptides, as disclosed in the present invention, it is within the scope of the present invention to determine an appropriate DNA sequence, which will code for the selected amino acid sequence. The appropriate DNA sequence can be produced by conventional and well-known methods of synthesizing DNA sequences. The DNA sequences so produced can then be cloned into appropriate cloning vehicles and used to transform an appropriate host cell to produce the recombinant synthetic peptide. All of the methodology referred to above is conventional and well-known to persons skill in the art.

The synthetic peptides, used according to the present invention, are substantially homologous in sequence to a part of the amino acid sequence of a human myelin basic protein or the h-MBP. By "a part of the amino acid sequence" it is meant that the synthetic sequence can be of any length provided that the amino acid sequence is long enough to function to neutralize anti-human myelin protein or anti-MBP but not of a length which would result in the prior art problems when the synthetic peptides are used for *in vivo* treatment of Multiple Sclerosis. The synthetic peptides can range in length from 8 amino acids to 25 amino acids. In a preferred embodiment of the present invention, the synthetic peptides can range in length from about 15 amino acid residues to about 21 amino acid residues.

According to a preferred embodiment, the peptide used in the present invention is selected from 8 amino acid residues to 25 amino acid residues taken from a continuous amino acid sequence within the sequence shown below:

SEQID NO: 1
His His Pro Ala Arg Thr Ala His Tyr Gly Ser Leu Pro Gln Lys
Ser His Gly Arg Thr Gln Asp Gln Asp Pro Val Val His Phe Phe
Lys Asn Ile Val Thr Pro Arg Thr Pro Pro Pro Ser Gln Gly Lys
Gly

Preferred synthetic peptides used in the present invention are selected from the group consisting of:

Peptide # 27 (SEQID NO:2):
His His Pro Ala Arg Thr Ala His Tyr Gly Ser Leu Pro Gln
Lys;

Peptide # 16 (SEQID NO: 3):
Ala Arg Thr Ala His Tyr Gly Ser Leu Pro Gln Lys Ser His
Gly;

Peptide # 21 (SEQID NO: 4):
Tyr Gly Ser Leu Pro Gln Lys Ser His Gly Arg Thr Gln Asp
Gln;

Peptide # 56 (SEQID NO: 5):
Lys Ser His Gly Arg Thr Gln Asp Gln Asp Pro Val Val His Phe
Phe Lys Asn Ile Val Thr;

Peptide # 12 (SEQID NO: 6):
Thr Gln Asp Gln Asp Pro Val Val His Phe Phe Lys Asn Ile Val
Thr Pro Arg; and

Peptide # 15 (SEQID NO: 7):
Lys Asn Ile Val Thr Pro Arg Thr Pro Pro Pro Ser Gln Gly Lys Gly.

The potential role of anti-MBP in the pathogenesis of MS continues to be explored. Increased anti-MBP titers in patients with active MS were initially reported by Panitch et al (Panitch, H.S., Hooper, C.S., and Johnson, K.P., Arch Neurol 37:206-209, 1980) who used a solid phase radioimmunoassay with guinea-pig MBP. Patients with acute MS relapses have usually increased anti-MBP predominantly in free form, while some patients in clinical remission may have undetectable anti-MBP levels. During the transition phase from an acute relapse to remission, titers of free anti-MBP progressively decrease over weeks or months, while bound fractions of the antibody rise as compared to their initial value. In other patients in remission, it is possible to observe low titers of free and bound anti-MBP, usually with a F/B ratio below unity, suggesting that anti-MBP neutralizing antibody(ies) are bound to anti-MBP. Occasionally, patients who fit the criteria of clinically definite MS or patients who had neuropathologically confirmed MS had undetectable anti-MBP during active phases of their disease. It is possible that such patients have antibodies to other myelin proteins. The absence of a specific antibody scenario does not negate the potential importance of anti-MBP in the mechanism of demyelination in the majority of MS patients.

Recently, an MBP antibody cascade was observed in the IgG fraction purified from MS CSF (Warren, K.G. and Catz, I., J Neurol Sci 96:19-27, 1990). Primary antibodies to MBP in both free and bound forms occur in association with active disease: F/B ratios are above unity in patients with acute relapses, and below unity in patients with chronic progressive disease (Warren, K.G. and Catz, I., Ann Neurol 209:20-25, 1986; Catz, I. and Warren, K.G., Can J Neurol Sci 13:21-24, 1986; and Warren, K.G. and Catz, I., Ann Neurol 21: 183-187, 1987). Secondary antibodies which neutralize anti-MBP appear when the disease becomes inactive. Tertiary antibodies which inhibit anti-MBP neutralization are present when the disease is chronically progressive and fails to become inactive. The fact that an MBP antibody cascade is associated with distinct phases of MS suggest its possible importance vis-a-vis the natural history of this illness.

Although anti-MBP can be detected in CSF of patients with active MS, their direct role in the pathogenesis of demyelination remains to be confirmed. The involvement of anti-MBP in the mechanism of MS could best be determined by their neutralization, *in vivo*, perhaps by administration of selected synthetic peptides and monitoring the clinical course of the disease. If anti-MBP is (are) the only primary antibody(ies) associated with demyelination in MS, it may be possible to block this process by intrathecal administration of selected synthetic MBP peptides which would neutralize anti-MBP and would promote tolerance to MBP in situ. Other human myelin proteins may also be involved with the demyelination in MS and accordingly, it is proposed to use synthetic peptides substantially homologous in sequence to a part of the amino acid sequence of these other myelin proteins to neutralize the corresponding antibodies. Although previous attempts to treat MS by intramuscular or subcutaneous administration of heterologous MBP have not been entirely successful (Campbell, B., Vogel, R.J., Fisher, E. and Lorenz, R., Arch Neurol 29:10-15, 1973; Gonsette, R.E., Delmotte, P. and Demonty, L. J Neurol 216:27-31, 1977; and Romine, J.S. and Salk, J., In: Hallpike, J.F., Adams, C.W.M. and Tourtelotte, W.W., eds. Multiple sclerosis. Baltimore. Williams & Wilkins, 1982:621-630), intrathecal administration of synthetic MBP peptides which neutralize anti-MBP is more likely to produce beneficial results.

The therapeutic dose for the treatment of MS may be from about 1.0 mg per kilogram of body weight to about 10.0 mg per kilogram of body weight. The synthetic peptides when admixed with a suitable pharmaceutically acceptable carrier can be administered intravenously, intrathecally or orally. It would be preferred to administer the peptides either intravenously or intrathecally.

While this invention is described in detail with particular reference to preferred embodiments thereof, the following examples, are offered to illustrate but not limit the invention.

### EXAMPLE

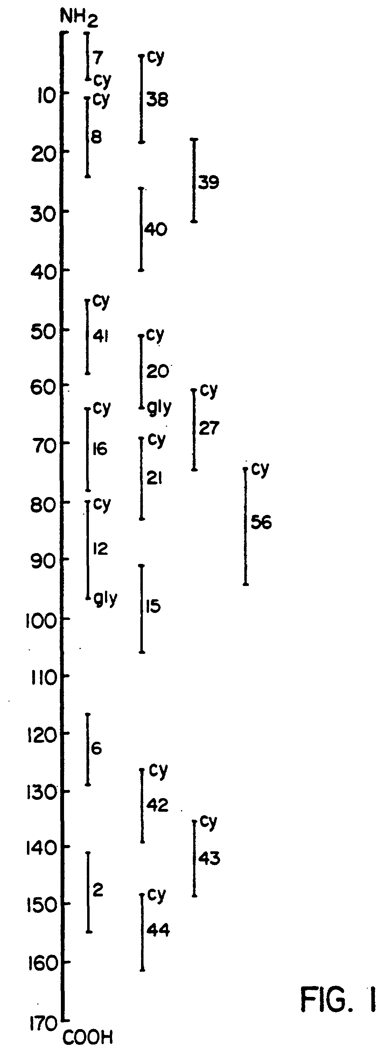

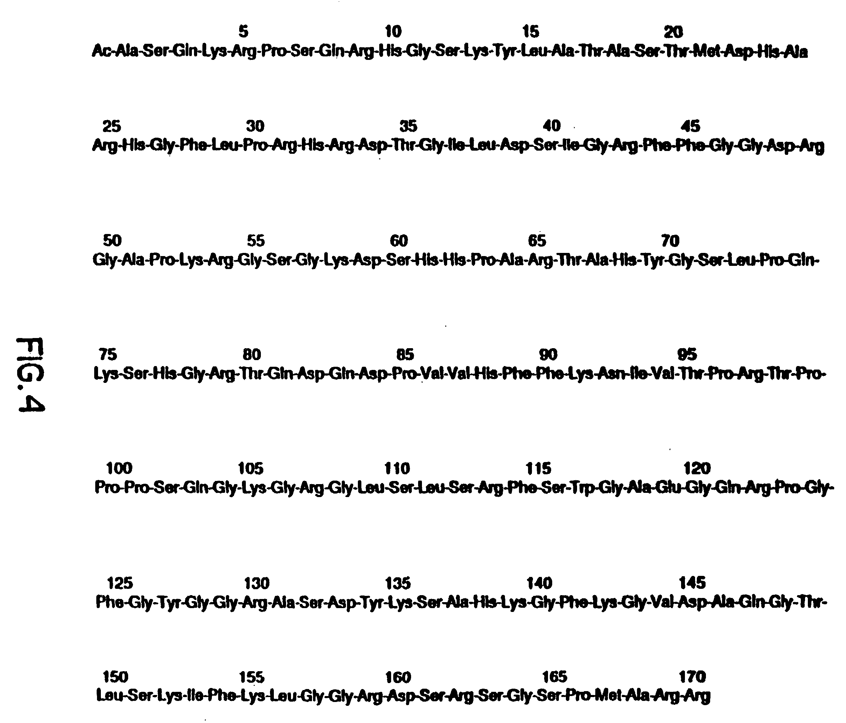

Figure 1 shows the localization of 18 synthetic peptides of h-MBP used in this study in relation to the intact MBP molecule. Figure 4 shows the complete amino acid sequence of the human myelin basic protein, shown schematically in Figure 1.

Native MBP was isolated from non-MS brain tissue (Diebler, G.E., Martenson, R.E., Kies, M.W., Prep Biochem 2:139-165, 1972) and further purified by gel filtration. The final antigen preparations were checked for purity by SDS-polyacrylamide gel electrophoresis. Only preparations that migrated at the molecular weight of 18.5 KD were used in further studies. Purified MBP was used in antigen-specific affinity chromatography, in neutralization studies and in the solid phase anti-MBP radioimmunoassay.

Eighteen synthetic peptides covering the length of h-MBP and containing between 8 and 25 amino acid residues were synthesized by the Fmoc method as previously described (Groome, N.P., Dawkes, A., Barry, R. et al. J Neuroimmun 19:305-315, 1988). Peptide purity was checked by reverse-phase high pressure liquid chromatography with a C18 column and water/acetonitrile gradient (0.1% TFA). Amino acid analysis of peptides was also performed using standard analysis. Many of the peptides used in this study contained an unnatural cysteine residue as they were made to function as immunogens. This is unlikely to affect the present findings.

Cerebrospinal fluid (CSF) was obtained within a week from the onset of symptoms from 35 patients with acute MS relapses and IgG levels were determined by nephelometry. CSF samples used in this study were selected to have initially high absolute IgG levels (≥ 0.80 g/l) and increased titers of anti-MBP (F/B ratio > 1.0). All MS patients had clinically definite disease.

IgG was purified from concentrated CSF of patients with acute MS by protein A-Sepharose (Pharmacia™) affinity chromatography as previously described (Warren, K.G. and Catz, I., J Neurol Sci 96:19-27, 1990). The purity of each IgG preparation was checked by polyacrylamide gel electrophoresis and isoelectric focusing. When elevated anti-MBP levels from purified IgG were absorbed to zero with MBP, the post-absorption supernatants contained residual IgG.

Purified MBP was coupled to CNBr-activated Sepharose 4B (Pharmacia™) according to the manufacturer's instructions. Purified CSF IgG containing increased anti-MBP levels from 35 patients with acute MS relapses was used as starting samples to isolate unbound anti-MBP by MBP-Sepharose affinity chromatography (Warren, K.G. and Catz, I., J Neurol Sci 103:90-96, 1991). Purified anti-MBP samples were compared with the initial IgG source by poly-acrylamide gel electrophoresis. When purified anti-MBP was absorbed to zero with MBP, the post-absorption supernatants contained no residual IgG.

Constant amounts of anti-MBP (15 radioactivity binding units corresponding to 100 for scale expansion purposes =%O) were incubated with increasing amounts of h-MBP (0-1000 ng) or individual synthetic peptides of MBP (0-10,000 ng) in a liquid phase assay and after 1.5 hours incubation, free anti-MBP levels were determined in all mixtures. Anti-MBP isolated from 7 individual MS patients or pooled anti-MBP from 10 different MS patients were used in neutralization experiments. Calf thymus histone and human serum albumin were used as negative antigen controls (range: 10-1000 ng). One monoclonal antibody to peptide #16 (clone 26) and one polyclonal rabbit antiserum to peptide #7 (R155) were used as positive antibody controls (Groome, N., Harland, J., and Dawkes, A., Neurochem Int 7:309-317, 1985; Barry, R., Payton, M., and Groome, N., Neurochem Int 2:291-300, 1991). Another mouse monoclonal antibody to epitope 45-50 (clone 16) was used as negative antibody control.

Anti-MBP levels were determined by a solid phase radioimmunoassay with human MBP (Warren, K.G. and Catz, I., Ann Neurol 209:20-25, 1986; Warren, K.G. and Catz, I., Ann Neurol 21:183-187, 1987; and Warren, K.G. and Catz, I., J Neurol Sci 91:143-151, 1989). Free anti-MBP levels were measured in all fractions from affinity chromatographies and in all neutralization mixtures. All individual samples were run in quadruplicate using the same iodinated material in order to minimize between-assay variability.

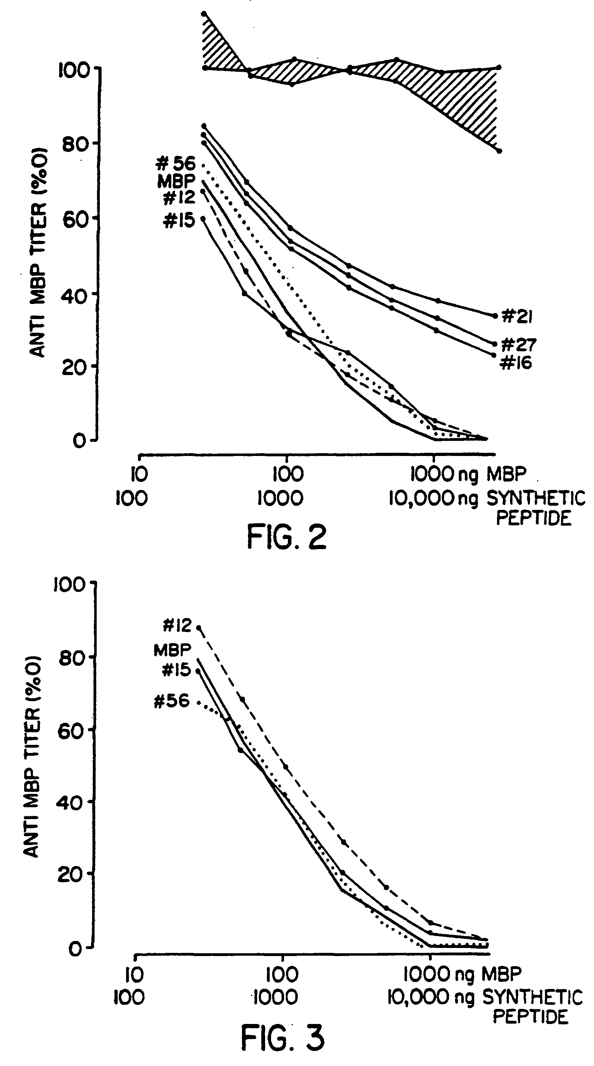

Purified anti-MBP was completely neutralized by MBP and by peptides #12 (residues: 80 - 97), #15 (residues: 91 - 106) and #56 (residues 75 - 95) and was partially neutralized by peptides #16 (residues 64 - 78), #21 (residues 69 - 83) and #27 (residues 61 - 75) (Table 1 and Figure 2). The remaining twelve synthetic peptides did not neutralize purified anti-MBP and their kinetic curves fell. within the grey area shown in Figure 2. Calf thymus histone and human serum albumin did not react with purified anti-MBP even at concentrations as high as 1000 ng. Clone 26 was only inhibited by peptide #16. R155 was only inhibited by peptide #7. Clone 16 did not react with MBP or any of the peptides (for clarity of the figure, control data are not illustrated). The control samples demonstrate the validity of the neutralization approach as each control antibody was neutralized completely by the expected peptide and by none of the other peptides. This shows that even the high peptide concentrations (10,000 ng) specificity of recognition was observed.

**TABLE 1**

| PEPTIDE NUMBER | HUMAN MBP SEQUENCE | | | REACTIVITY WITH ANTI-MBP |
|---|---|---|---|---|
| 7 | | 1 - 8 | Cy | - |
| 38 | Cy | 4 - 18 | | - |
| 8 | Cy | 11 - 24 | | - |
| 39 | | 18 - 32 | | - |
| 40 | | 26 - 40 | | - |
| 41 | Cy | 35 - 58 | | - |
| 20 | Cy | 51 - 64 | Gly | - |
| 16 | Cy | 64 - 78 | | + |
| 27 | Cy | 61 - 75 | | + |
| 21 | Cy | 69 - 83 | | + |
| 56 | Cy | 75 - 95 | | ++ |
| 12 | Cy | 80 - 97 | Gly | ++ |
| 15 | Cy | 91 - 106 | | ++ |
| 6 | | 117 - 129 | | - |
| 42 | Cy | 127 - 140 | | - |
| 43 | Cy | 136 - 149 | | - |
| 2 | | 141 - 155 | | - |
| 44 | Cy | 149 - 162 | | - |
| ++ complete neutralization | | | | |
| + partial neutralization | | | | |
| - insignificant reactivity | | | | |

Anti-MBP purified from 7 individual MS patients was completely neutralized by h-MBP and peptides #12, #15 and #56 (see Figure 3 as an illustrative example). Due to the limited amount of antibody obtained from individual MS patients, anti-MBP was not reacted with the remaining 15 synthetic peptides.

As noted previously, anti-MBP was neutralized with synthetic peptides spanning from about amino acid residue 61 to about amino acid residue 106. The synthetic peptides which did not neutralize anti-MBP cover both the amino ( about residues 1 to 63) and the carboxyl (about residues 117 to 162) terminals of h-MBP. It appears that residues 117 to 162) terminals of h-MBP. It appears that peptides from different non-overlapping regions of MBP neutralize the same antibody(ies). This might be explained if the antibodies recognize a discontinuous (assembled) epitope containing amino acids from different regions. A similar phenomenon has been previously observed by Hruby et al (Hruby, S., Alvord, E.C., Groome, N.P. et al, Molec Immun 24: 1359-1364, 1987) who showed that a rat monoclonal antibody had a major epitope in MBP sequence 112-121 but a strong cross-reaction with another epitope in peptide 39-91. This is more likely than the possibility that the antibody is cross-reactive with two completely different sequences which did not form a discontinuous epitope (Hruby, S., Alvord, E.C., Martenson, R.E., et al. J Neurochem 44:637-650, 1985). The neutralization data could be explained by the ability of peptides from different sections of MBP to each partially occupy the antibody binding pocket by interacting with different antibody amino acid side chains. This explanation fits the observation that the peptides giving complete inhibition (#12, #15 and #56) are approximately 100 times less effective on a molar basis than intact MBP at causing inhibition. By the hypothesis advanced above, this could be due to each peptide clone being unable to achieve the binding energy of the original MBP epitope.

## Claims

1. Use of a peptide having a length of from 8 amino acids to 25 amino acids, which peptide is substantially homologous in sequence to the amino acid sequence of a human myelin basic protein from amino acid 61 to amino acid 106 and is able to neutralize anti-myelin basic protein, in the manufacture of a medicament for the treatment of multiple sclerosis in humans, which medicament comprises the peptide as an active ingredient.

2. Use according to claim 1, wherein the peptide has a length of from 15 amino acids to 21 amino acids.

3. Use according to claim 1 or claim 2, wherein the peptide is selected from peptides having from 8 consecutive amino acid residues to 25 consecutive amino acid residues within the sequence SEQID NO:1.

4. Use according to claim 3, wherein the peptide is selected from the group consisting of SEQID NO:2, SEQID NO: 3, SEQID NO:4, SEQID NO:5, SEQID NO:6, AND SEQID NO: 7.

5. A process for the manufacture of a medicament for the treatment of multiple sclerosis in a human, wherein said process comprises admixing a peptide having a length of from 8 amino acids to 25 amino acids, which peptide is substantially homologous in sequence to the amino acid sequence of a human myelin basic protein from amino acid 61 to amino acid 106 and is able to neutralize anti-myelin basic protein, with a suitable pharmaceutically acceptable carrier.

6. The process according to claim 5, wherein the peptide has a length of from 15 amino acids to 21 amino acids.

7. The process according to claim 5 or claim 6, wherein the peptide is selected from peptides having from 8 consecutive amino acid residues to 25 consecutive amino acid residues within the sequence SEQID NO:1.

8. The process according to claim 7, wherein the peptide is selected from the group consisting of SEQID NO: 2, SEQID NO:3, SEQID NO: 4, SEQID NO: 5, SEQID NO: 6, AND SEQID NO: 7.

9. The process according to any one of claims 5 to 8, wherein the pharmaceutical composition is suitable for administration intravenously or intrathecally or orally, especially intravenously or intrathecally.

## Patentansprüche

1. Verwendung eines Peptids einer Länge von 8 bis 25 Aminosäuren, das in seiner Sequenz mit der Aminosäure-Sequenz eines humanen basischen Myelinproteins von Aminosäure 61 bis Aminosäure 106 im Wesentlichen homolog ist und anti-basisches Myelinprotein neutralisieren kann, für die Herstellung eines Medikaments für die Behandlung von multipler Sklerose bei Menschen, wobei das Medikament das Peptid als einen aktiven Bestandteil umfasst.

2. Verwendung nach Anspruch 1, wobei das Peptid 15 bis 21 Aminosäuren lang ist.

3. Verwendung nach Anspruch 1 oder 2, wobei das Peptid ausgewählt ist aus Peptiden, die 8 bis 25 aufeinanderfolgende Aminosäuren aus der Sequenz SEQ ID NR: 1 aufweisen.

4. Verwendung nach Anspruch 3, wobei das Peptid ausgewählt ist aus der aus den SEQ ID NRn: 2, 3, 4, 5, 6 und 7 bestehenden Gruppe.

5. Verfahren zur Herstellung eines Medikaments für die Behandlung von multipler Sklerose bei Menschen, wobei dieses Verfahren den Schritt umfasst, ein Peptid mit einer Länge von 8 bis 25 Aminosäuren mit einem geeigneten pharmazeutisch verträglichen Träger zu mischen, wobei das Peptid in seiner Sequenz im Wesentlichen homolog mit der Aminosäure-Sequenz eines humanen basischen Myelinproteins von Aminosäure 61 bis Aminosäure 106 ist und fähig ist, anti-basisches Myelinprotein zu neutralisieren.

6. Das Verfahren nach Anspruch 5, wobei das Peptid 15 bis 21 Aminosäuren lang ist.

7. Das Verfahren nach Anspruch 5 oder 6, wobei das Peptid ausgewählt ist aus Peptiden, die 8 bis 25 aufeinanderfolgende Aminosäuren aus der Sequenz SEQ ID NR: 1 aufweisen.

8. Das Verfahren nach Anspruch 7, wobei das Peptid ausgewählt ist aus der aus den SEQ ID NRn: 2, 3, 4, 5, 6 und 7 bestehenden Gruppe.

9. Das Verfahren nach einem der Ansprüche 5 bis 8, wobei die pharmazeutische Zusammensetzung geeignet ist für die intravenöse, die intrathekale oder die orale Verabreichung, insbesondere für die intravenöse oder intrathekale Verabreichung.

## Revendications

1. Utilisation d'un peptide ayant une longueur de 8 aminoacides à 25 aminoacides, peptide qui possède une séquence essentiellement analogue à la séquence d'aminoacides d'une protéine basique de myéline humaine de l'aminoacide 61 à l'aminoacide 106 et qui est capable de neutraliser une protéine basique anti-myéline, dans la fabrication d'un médicament pour le traitement de la sclérose en plaques chez l'homme, médicament qui comprend le peptide en tant qu'ingrédient actif.

2. Utilisation selon la revendication 1, dans laquelle le peptide a une longueur de 15 aminoacides à 21 aminoacides.

3. Utilisation selon la revendication 1 ou la revendication 2, dans laquelle le peptide est choisi parmi les peptides ayant de 8 résidus d'aminoacide consécutifs à 25 résidus d'aminoacide consécutifs au sein de la séquence SEQID N°: 1.

4. Utilisation selon la revendication 3, dans laquelle le peptide est choisi dans le groupe constitué par SEQID N°: 2, SEQID N°: 3, SEQID N°: 4, SEQID N°: 5, SEQID N°: 6 et SEQID N°: 7.

5. Procédé de fabrication d'un médicament pour le traitement de la sclérose en plaques chez l'homme, dans lequel ledit procédé comprend le mélange d'un peptide ayant une longueur de 8 aminoacides à 25 aminoacides, peptide qui possède une séquence essentiellement analogue à la séquence d'aminoacides d'une protéine basique de myéline humaine de l'aminoacide 61 à l'aminoacide 106 et qui est capable de neutraliser une protéine basique anti-myéline, avec un véhicule convenable acceptable au plan pharmaceutique.

6. Procédé selon la revendication 5, dans lequel le peptide a une longueur de 15 aminoacides à 21 aminoacides.

7. Procédé selon la revendication 5 ou la revendication 6, dans lequel le peptide est choisi parmi les peptides ayant de 8 résidus d'aminoacide consécutifs à 25 résidus d'aminoacide consécutifs au sein de la séquence SEQID N°: 1.

8. Procédé selon la revendication 7, dans lequel le peptide est choisi dans le groupe constitué par SEQID N°: 2, SEQID N°: 3, SEQID N°: 4, SEQID N°: 5, SEQID N°: 6 et SEQID N°: 7.

9. Procédé selon l'une quelconque des revendications 5 à 8, dans lequel la composition pharmaceutique convient à une administration par voie intraveineuse ou intrathécale ou par voie orale, en particulier par voie intraveineuse ou intrathécale.
